# EUROPEAN PATENT APPLICATION

(11) **EP 4 769 910 A1**
(43) Date of publication of application: **01.07.2026**
(21) Application number: 24870307.6
(22) Date of filing: 29.08.2024
(51) Int. Cl.: H02K 7/10, A61B 34/30, H02K 11/20, H02K 11/215

(54) **INSTRUMENT DRIVER AND SURGICAL ROBOT**

(30) Priority: 27.09.2023 CN 202311283181; 27.09.2023 CN 202322668771 U
(71) Applicant: Cornerstone Technology (Shenzhen) Limited, Shenzhen, Guandong 518066 (CN)
(72) Inventor: REN, Lixue, Shenzhen, Guandong 518066 (CN); LI, Shengguang, Shenzhen, Guandong 518066 (CN)
(74) Representative: Balder IP Law, S.L.
(86) International application number: PCT/CN2024/115379
(87) International publication number: WO 2025/066761

(57) **Abstract**

An instrument drive and a surgical robot are disclosed. The instrument drive includes a carrier, a first circuit board, an actuating assembly, a drive output assembly, and a first encoder. The carrier has an opening. The first circuit board covers the opening and is fixed to the carrier, and defines a through hole. The actuating assembly includes a reducer output shaft, and a portion of the reducer output shaft passes through the through hole. The drive output assembly is coupled to the reducer output shaft. The first encoder includes a first encoder rotating portion and a first encoder stationary portion, the first encoder stationary portion being at least a part of the first circuit board. At least one of the drive output assembly, the first encoder rotating portion, and the reducer output shaft is provided with a dynamic seal structure coupled to the first circuit board.

## Description

### CROSS-REFERENCE TO RELATED APPLICATIONS

The present application claims priority to Chinese Patent Application No. 202311283181.9, filed on September 27, 2023, entitled "Instrument drive and Surgical Robot", and Chinese Patent Application No. 202322668771.5, filed on September 27, 2023, entitled "Instrument drive and Surgical Robot", the entire contents of which are incorporated herein by reference.

### TECHNICAL FIELD

The present disclosure relates to the field of surgical robots, and in particular to an instrument drive and a surgical robot.

### BACKGROUND

Medical surgical robots have advantages such as high positioning accuracy, stable operation, strong dexterity, a large working range, and resistance to radiation and infection, and thus have been widely applied in various surgeries. Use of the medical surgical robots may improve surgical accuracy, mitigate hand tremor and fatigue of surgeons, and compensate for neuromuscular feedback, thereby enabling the surgeons to perform surgical operations in a more comfortable state. Such robots are of great value for improving surgical success rates and reducing patient pain, and thus research on the robots has become a new field of medical device applications in recent years.

The surgical robot is typically provided with surgical instrument drive/output components at ends of one or more robotic arms thereof, to satisfy needs of changing and adapting various surgical instruments for different types of surgeries and throughout the entire surgical procedure, thereby performing various actions in the surgery. In other words, an instrument drive is used for supporting, engaging with, and driving a robotic arm of a surgical robot. Since thededicated robotic arm of the surgical robot typically has multiple (four to six) degrees of freedom, the instrument drive needs to be equipped with a corresponding number of drive units to actuate the robotic arm. Each drive unit independently includes components such as a motor, an encoder, and a reduction gearbox.

However, inventors have found that, in some situations, external impurities may affect the above components, thereby affecting motion accuracy of the instrument drive and the surgical robot.

### SUMMARY

According to a first aspect of embodiments of the present disclosure, an instrument drive applicable to a surgical robot is provided, the instrument drive includes a carrier, a first circuit board, an actuating assembly, a drive output assembly, and a first encoder. The carrier defining an opening, the first circuit board covering the opening and fixed to the carrier, the first circuit board and the carrier enclosing a receiving space; the first circuit board defining a through hole communicating the receiving space with an external environment. The actuating assembly including a reducer output shaft, the reducer output shaft is partly disposed in the receiving space and extends out of the receiving space via the through hole, the reducer output shaft being rotatable about an axis of the reducer output shaft. The drive output assembly coupled to the reducer output shaft, the drive output assembly being rotatable with the reducer output shaft about the axis of the reducer output shaft, the drive output assembly is disposed at an end of the reducer output shaft away from the receiving space. The first encoder includes a first encoder rotating portion and a first encoder stationary portion. The first encoder rotating portion fixed to the reducer output shaft, the first encoder rotating portion being rotatable with the reducer output shaft about the axis of the reducer output shaft, the first encoder rotating portion is disposed in the receiving space. The first encoder stationary portion being at least a part of the first circuit board, the first encoder stationary portion configured to cooperate with the first encoder rotating portion to detect rotation of the drive output assembly. Wherein one or more of the drive output assembly, the first encoder rotating portion, and the reducer output shaft is provided with a dynamic seal structure coupled to the first circuit board.

In some embodiments, the drive output assembly is coupled to a first sealing member, the first sealing member surrounds the drive output assembly and is fixedly embedded in an end of the drive output assembly proximate to the receiving space, an orthographic projection of the first sealing member on the first circuit board surrounds the through hole, the first sealing member abuts against a surface of the first circuit board facing away from the receiving space, and the first sealing member is kept abutting against the first circuit board in rotation of the first sealing member.

In some embodiments, the first encoder rotating portion is coupled to a second sealing member, the second sealing member surrounds the first encoder rotating portion and is fixedly embedded in an end of the first encoder rotating portion proximate to the first circuit board. The second sealing member abuts against the first circuit board, the second sealing member surrounds an outer circumference of the reducer output shaft, and an orthographic projection of the second sealing member onto the first circuit board surrounds the through hole, and the second sealing member is kept abutting against the first circuit board in rotation of the second sealing member.

In some embodiments, the first circuit board is coupled to a third sealing member, the third sealing member is disposed in the through hole, the third sealing member is disposed in a gap between the reducer output shaft and the first circuit board and surrounds the reducer output shaft, and an inner sidewall of the third sealing member abuts against the reducer output shaft.

In some embodiments, the third sealing member includes an outer ring portion and an inner ring portion, an outer circumference of the outer ring portion defines a groove, an inner rim of the first circuit board surrounding the through hole is embedded in the groove, the outer ring portion covers the inner rim of the first circuit board surrounding the through hole, the inner ring portion defines a hole, the reducer output shaft passes through the hole, and a diameter of the hole is equal to or less than an outer diameter of the reducer output shaft.

In some embodiments, a thickness of the inner ring portion is less than a width of the groove, or the thickness of the inner ring portion is less than a thickness of the inner rim of the first circuit board surrounding the through hole.

In some embodiments, an orthographic projection of the first encoder rotating portion onto the first circuit board is larger than the through hole.

In some embodiments, the first encoder is a photoelectric encoder, the first encoder rotating portion includes at least a photoelectric code disk, and an orthographic projection of the photoelectric code disk on the first circuit board is larger than the through hole.

In some embodiments, the first encoder is a photoelectric encoder, the first encoder rotating portion includes at least a photoelectric code disk, and the encoder stationary portion at least includes a photosensitive element. The photoelectric code disk is disposed in the receiving space and fixedly coupled to the reducer output shaft, and a light source and the photosensitive element are provided on the first circuit board.

In some embodiments, the instrument drive includes a plurality of actuating assemblies, a plurality of drive output assemblies, and a plurality of first encoders. The plurality of actuating assemblies, the plurality of drive output assemblies, and the plurality of first encoders are equal in number and correspond one-to-one, the first circuit board defining a plurality of through holes, and the plurality of through holes correspond to the plurality of first encoders one-to-one

In some embodiments, the drive output assembly includes a guide disk and a drive disk. The guide disk fixed to the reducer output shaft and configured to move synchronously with the reducer output shaft; the sealing member is embedded in the guide disk. The drive disk slidably coupled to the drive disk along an axial direction of the reducer output shaft, the drive disk and the guide disk being synchronously movable in a radial direction.

In some embodiments, the drive disk is axially movable between a first position and a second position, the first position is closer to the receiving space than the second position. When the drive disk is at the first position, a minimum distance from the drive disk to the first circuit board is not less than an axial dimension of the sealing member.

In some embodiments, the instrument drive further includes an actuator and a second encoder. The actuator coupled to a reduction gearbox and configured to actuate the reducer output shaft to rotate. The second encoder including a second encoder rotating portion and a second encoder stationary portion, the second encoder rotating portion is coupled to the actuator and actuated by the actuator to rotate, the second encoder stationary portion is fixed relative to the carrier, and the second encoder stationary portion cooperates with the second encoder rotating portion to detect rotation of an output portion of the actuator.

In some embodiments, the actuator is a motor, the motor includes a motor output shaft fixed to an input portion of the reduction gearbox and coaxial with the input portion of the reduction gearbox, and the second encoder is configured to detect rotation of the motor output shaft.

In some embodiments, a rotation axis of the motor output shaft is colinear with a rotation axis of the reducer output shaft, and a rotation axis of the first encoder rotating portion is colinear with a rotation axis of the second encoder rotating portion.

In some embodiments, the second encoder is a magnetoelectric encoder, the second encoder rotating portion including at least a magnetic element, the second encoder stationary portion including at least a magnetosensitive element, and the second encoder stationary portion is spaced apart from the second encoder rotating portion along the rotation axis of the second encoder rotating portion.

In some embodiments, the instrument drive further includes a second circuit board fixedly provided on the carrier, and the second encoder stationary portion is fixedly disposed on the second circuit board.

In some embodiments, the drive output assembly covers an end of the reducer output shaft facing away from the receiving space.

According to a second aspect of embodiments of the present disclosure, a surgical robot is provided, the surgical robot including the instrument drive described above.

### BRIEF DESCRIPTION OF THE DRAWINGS

To more clearly illustrate the technical solutions in the embodiments of the present disclosure, the drawings needed in the description of the embodiments are briefly introduced below. Apparently, the drawings in the following description are only some embodiments of the present disclosure, and a person of ordinary skill in the art can obtain other drawings based on these drawings without creative effort.
FIG. 1 is a schematic structural diagram of a surgical robot according to one embodiment of the present disclosure.
FIG. 2 is another schematic diagram of the surgical robot according to one embodiment of the present disclosure.
FIG. 3 is a schematic diagram of an instrument drive according to one embodiment of the present disclosure.
FIG. 4 is another schematic diagram of the instrument drive according to one embodiment of the present disclosure.
FIG. 5 is a schematic partial diagram of the instrument drive according to one embodiment of the present disclosure.
FIG. 6 is another schematic partial diagram of the instrument drive according to one embodiment of the present disclosure.
FIG. 7 is a schematic diagram of a first encoder in the instrument drive according to one embodiment of the present disclosure.
FIG. 8 is a schematic diagram of a second encoder in the instrument drive according to one embodiment of the present disclosure.
FIG. 9 is a schematic diagram of an instrument drive according to another embodiment of the present disclosure.
FIG. 10 is a schematic diagram of an instrument drive according to yet another embodiment of the present disclosure.
FIG. 11 is an enlarged view of region A in FIG. 10.

### DETAILED DESCRIPTION OF THE EMBODIMENTS

Example embodiments are described in detail herein, and examples thereof are shown in the drawings. In the following description, unless otherwise indicated, like numerals in different drawings denote the same or similar elements. The implementations described in the following example embodiments do not represent all implementations consistent with the present disclosure. Rather, they are merely examples of devices and methods consistent with some aspects of the present disclosure as detailed in the appended claims.

The terminology used in the present disclosure is for the purpose of describing particular embodiments only and is not intended to limit the present disclosure. Unless otherwise defined, technical or scientific terms used herein have the meanings commonly understood by those of ordinary skill in the art to which the present disclosure pertains. The terms "a" or "an" and similar words used in the specification and the appended claims do not denote a limitation of quantity, but rather denote the existence of at least one. The term "plurality" means two or more. The terms "include" or "comprise" and similar words indicate that elements or objects listed after "include" or "comprise" and their equivalents are encompassed, and do not exclude other elements or objects. The terms "connect" or "couple" and similar words are not limited to physical or mechanical connections, and may include electrical connections, whether direct or indirect. The terms "upper" and/or "lower" and similar words are used for convenience of description only, and are not limited to a position or a spatial orientation. The singular forms "a", "said", and "the" used in the specification and the appended claims are also intended to include plural forms unless the context clearly indicates otherwise. It should also be understood that the term "and/or" as used herein refers to and includes any and all possible combinations of one or more of the associated listed items.

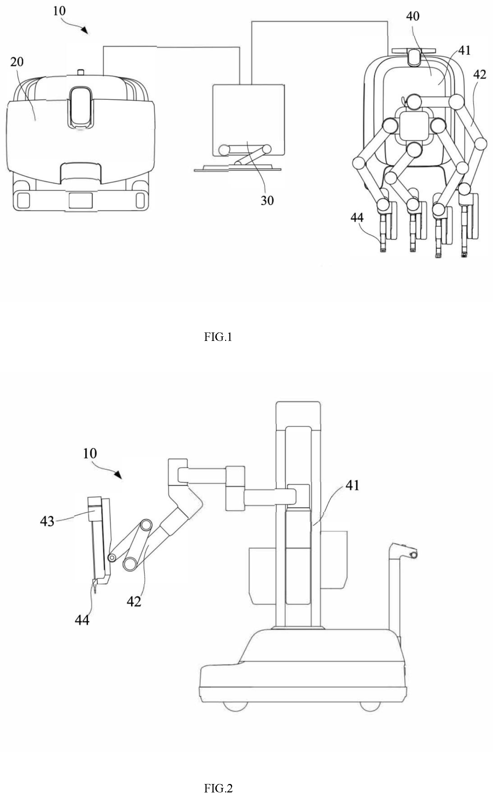

With reference to FIGS. 1-8, exemplary structures of a surgical robot and an instrument drive applied thereto are described below.

Referring to FIGS. 1 and 2, the surgical robot 10 is disclosed. The surgical robot 10 includes a console 20, an imaging system 30, and a robotic arm system 40. The robotic arm system 40 includes a base 41, an operating arm 42, and an instrument drive 43. The operating arm 42 and the instrument drive are disposed on the base 41, and the base 41 may move the components thereon as a whole to a peripheral side of a patient. The operating arm 42 is configured to couple to a suitable surgical instrument 44, and the instrument drive 43 is coupled to the operating arm 42 to control motion of the operating arm 42. In actual operation, a physician may control the instrument drive 43 in the robotic arm system 40 disposed around the patient via the console 20, so as to control the operating arm 42 and the surgical instrument by means of the instrument drive 43. A camera module (not shown) may also be fixed on the operating arm 42. The camera module may follow the surgical instrument 44 into a corresponding position in the patient and acquire image information of that position, and the imaging system 30 may display the acquired image information to assist the physician in performing the surgical operation.

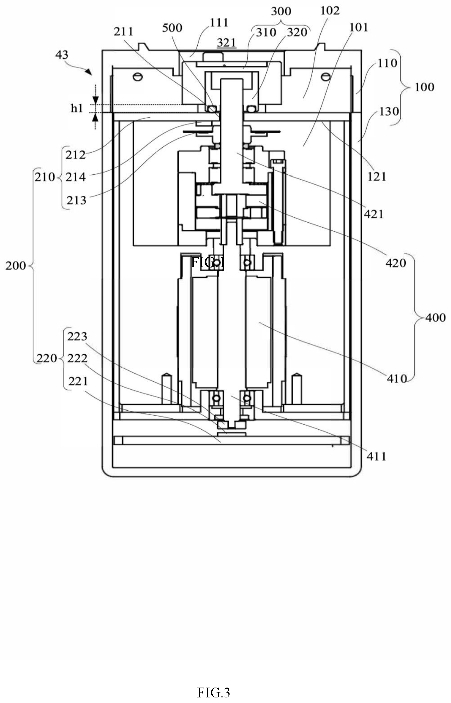

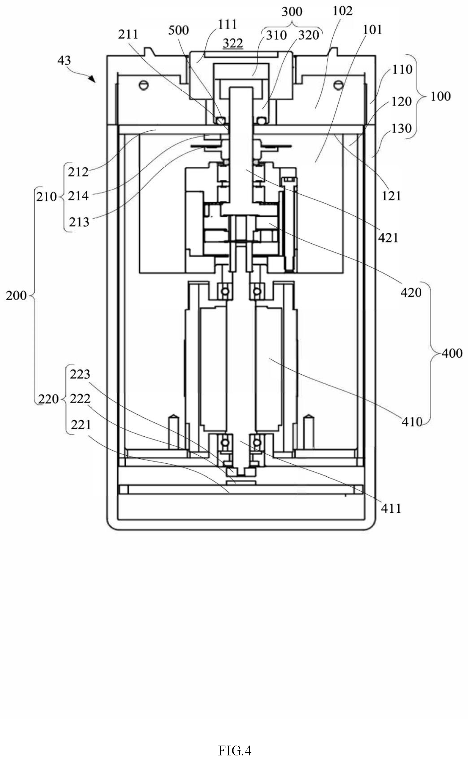

Specifically, as shown in FIGS. 3 and 4, and with reference to FIGS. 1 and 2 when necessary, the instrument drive 43 includes a housing assembly 100, an encoder assembly 200, a drive output assembly 300, and an actuating assembly 400. One end of the drive output assembly 300 is fixed to the actuating assembly 400, and the other end is configured to couple the surgical instrument 44. When the actuating assembly 400 operates, the drive output assembly 300 may rotate driven by the actuating assembly 400, and drive the surgical instrument 44 to perform corresponding actions. The encoder assembly 200 is arranged at the actuating assembly 400 to detect rotation output accuracy of the actuating assembly 400, thereby ensuring motion accuracy of the surgical instrument 44. The encoder assembly 200, the drive output assembly 300, and the actuating assembly 400 are all arranged in the housing assembly 100. The housing assembly 100 defines a receiving space 101, and may support and protect components disposed in the receiving space 101.

Referring to FIGS. 3 and 4, the actuating assembly 400 may include an actuator and a reducer 420, with the actuator coupled to the reducer 420. A reducer output shaft 421 may serve as an output member of the actuating assembly 400, and the actuator may actuate the reducer output shaft 421 to rotate. The actuator may be implemented as a motor 410. The motor 410 includes a motor output shaft 411, which is coaxially fixed to an input portion of a reduction gearbox. In other words, the motor output shaft 411 is fixed to an input end of the reducer 420 through which speed reduction occurs. The reducer 420 includes the reducer output shaft 421, and the drive output assembly 300 is coupled to the reducer output shaft 421 such that the drive output assembly 300 rotates at a suitable speed.

The encoder assembly 200 may include a stationary portion and a rotating portion. The stationary portion is configured as a portion fixed relative to the housing assembly 100, and the rotating portion is configured as a portion rotatable when actuated by the motor. The encoder assembly 200 may include a first encoder 210. The first encoder 210 includes a first encoder stationary portion and a first encoder rotating portion. The first encoder stationary portion cooperates with the first encoder rotating portion to detect rotation of the drive output assembly. The first encoder rotating portion is fixed to the reducer output shaft 421 and being rotatable with the reducer output shaft 421 about an axis of the reducer output shaft 421. The first encoder rotating portion is disposed in the receiving space 101 and has an axisymmetric structure about an axis of the reducer output shaft 421. The first encoder stationary portion is fixed relative to the housing assembly and is configured to detect instantaneous or continuous state, such as rotational speed, angular velocity, and angle, of the reducer output shaft 421.

In one embodiment, the housing assembly 100 may include an upper housing 110, a lower housing 130, a carrier 120 fixed to the lower housing 130, and a first circuit board. The carrier 120 has an opening 121. The first circuit board covers the opening 121 and is fixed to the carrier 120, and the first circuit board and the carrier 120 together define the receiving space 101. The first circuit board defines a through hole that communicates the receiving space to an external environment. A cover structure formed by the first circuit board 212 covers the opening 121. The first encoder stationary portion may be at least part of the first circuit board. The first circuit board 212 is fixed to the carrier by screws. The first circuit board 212 and the carrier 120 together enclose the receiving space 101. Most of the structures of the motor 410 and the reducer 420 may be disposed in the receiving space 101, or only part of the structure of the reducer may be disposed in the receiving space. The upper housing 110 and the first circuit board 212 together define a transmission space 102. At least part of the drive output assembly 300 is disposed in the transmission space 102.

In the above configuration, the first circuit board 212 defines the through hole 211 that communicates the receiving space 101 and the transmission space 102. The reducer output shaft 421 is partly disposed in the receiving space 101 and extends out of the receiving space 101 via the through hole 211, and further extends into the transmission space 102 to couple with the drive output assembly 300 disposed in the transmission space 102 and to actuate the drive output assembly 300. The reducer output shaft 421 is rotatable about an axis of the reducer output shaft 421. The drive output assembly 300 is sleeved on and fixed to an outer circumference of the portion of the reducer output shaft 421 away from the receiving space 101.

The instrument drive 43 further includes a first sealing member 500. The first sealing member 500 surrounds the drive output assembly 300 and is embedded in an end of the drive output assembly 300 proximate to the receiving space 101. The first sealing member 500 also surrounds an outer circumference of the reducer output shaft 421, and an orthographic projection of the first sealing member 500 onto the first circuit board 212 surrounds the through hole 211. The first sealing member 500 abuts against a surface of the first circuit board 212 away from the receiving space 101 to limit impurities from entering the receiving space 101 through the through hole 211. The first sealing member 500 may be fixed to the drive output assembly 300 such that the first sealing member 500 rotates with the reducer output shaft 421 about an axis of the reducer shaft 421, and the first sealing member 500 is kept abutting against the first circuit board 212 to form the cover structure in rotation of the first sealing member 500; alternatively, the first sealing member 500 may form a sealing effect only by being compressed between the drive output assembly 300 and the first circuit board 212, without being fixed to either the drive output assembly 300 or the first circuit board 212.

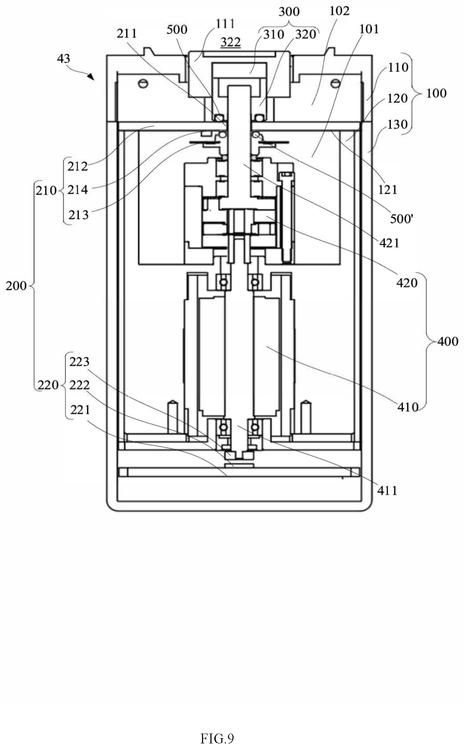

As shown in FIG. 9, in another embodiment, the instrument drive 43 may further include a second sealing member 500'. The first encoder rotating portion is fixed to the second sealing member 500'. The second sealing member 500' surrounds the first encoder rotating portion and is embedded in an end of the first encoder rotating portion near the first circuit board and abuts against the first circuit board. The second sealing member 500' surrounds an outer circumference of the reducer output shaft 421, and an orthographic projection of the second sealing member 500' onto the first circuit board surrounds the through hole 211, and the second sealing member 500' is rotatable with the reducer output shaft 421 about an axis of the reducer output shaft 421. Alternatively, the second sealing member 500' may form a sealing effect only by being compressed between the first encoder rotating portion and the first circuit board, without being fixed to either the first encoder rotating portion or the first circuit board. In any case, when the second sealing member 500' rotates, the second sealing member 500' is kept abutting against the first circuit board 212.

It should be noted that only the first sealing member 500 may be provided in the instrument drive, or only the second sealing member 500' may be provided, or both the first sealing member 500 and the second sealing member 500' may be provided.

An orthographic projection of the first encoder rotating portion onto the first circuit board is larger than the through hole 211. With this arrangement, the second sealing member 500' may be stably arranged on the first encoder rotating portion.

In the above configuration, the reducer output shaft 421 passes through the through hole 211 in the first circuit board 212, with one portion disposed in the receiving space 101 and another portion disposed in the transmission space 102. In a case that no sealing structure is provided, external impurities may enter the receiving space 101 through the transmission space 102 and through a gap between an inner wall of the through hole 211 and the reducer output shaft 421, thereby affecting operating stability of components disposed in the receiving space 101. In the present embodiment, the first sealing member 500 and/or the second sealing member 500' is provided between the drive output assembly 300 and the first circuit board 212. One end of the first sealing member 500 is embedded in the drive output assembly 300 to form a sealed connection to the drive output assembly 300, and another end of the first sealing member 500 abuts against the first circuit board 212 to form a dynamically sealed connection to the first encoder 210, thereby reducing or preventing external impurities from entering the receiving space 101 through the gap between the inner wall of the through hole 211 and the reducer output shaft 421. This arrangement helps improve operating stability of components in the receiving space 101 (including but not limited to the motor 410, the reducer 420, and the encoder assembly 200), thereby improving motion accuracy of the instrument drive 43 and the surgical robot 10 and ensuring reliability of surgery.

It should be noted that the instrument drive 43 may include a plurality of actuating assemblies 400, a plurality of drive output assemblies 300, and a plurality of first encoders 210. The plurality of actuating assemblies 400, the plurality of drive output assemblies 300, and the plurality of first encoders 210 are equal in number and correspond one-to-one. The first circuit board defines a plurality of through holes 211, and the plurality of through holes 211 correspond to the plurality of first encoders 210 one-to-one.

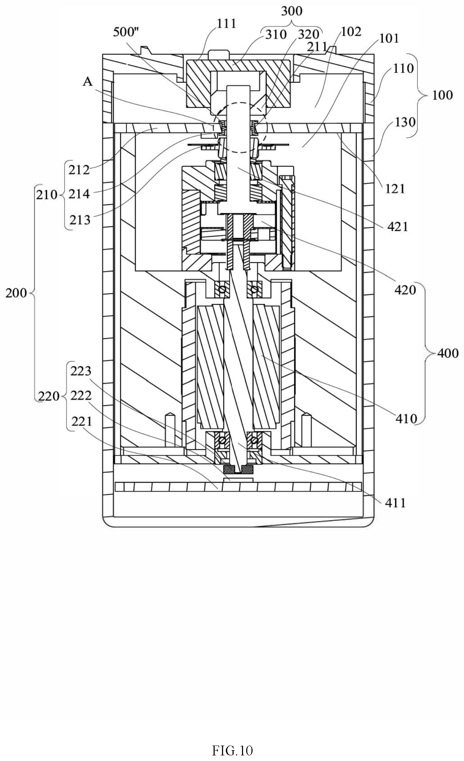

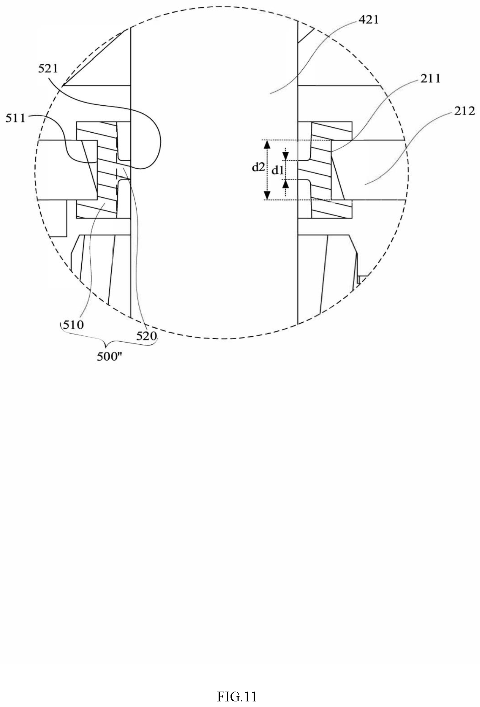

Referring to FIGS. 10 and 11, in another embodiment, the instrument drive 43 may further include a third sealing member 500", the first circuit board 212 is coupled with the third sealing member 500". Specifically, the third sealing member 500" is disposed in the through hole 211 of the first circuit board 212. The third sealing member 500" is disposed in a gap between the reducer output shaft 421 and the first circuit board 212. The third sealing member 500" surrounds the reducer output shaft 421. An inner sidewall of the third sealing member 500" abuts against the reducer output shaft 421. An outer sidewall of the third sealing member 500" abuts against the first circuit board 212.

By providing the third sealing member 500" in the through hole 211, external impurities are reduced or prevented from entering the receiving space 101 through the gap between the inner wall of the through hole 211 and the reducer output shaft 421, i.e., reduced or prevented from entering the receiving space 101 through the through hole 211. This helps improve operating stability of components in the receiving space 101 (including but not limited to the motor 410, the reducer 420, and the encoder assembly 200), thereby improving motion accuracy of the instrument drive 43 and the surgical robot 10 and ensuring reliability of surgery.

In one embodiment, the third sealing member 500" includes an outer ring portion 510 and an inner ring portion 520. An outer circumference of the outer ring portion 510 defines a groove 511, and an inner rim of the first circuit board 212 at the through hole 211 is embedded in the groove 511, such that the outer ring portion 510 covers the inner rim of the first circuit board 212 surrounding the through hole 211. The inner ring portion 520 defines a hole 521, and the reducer output shaft 421 passes through the hole 521, and a diameter of the hole 521 is equal to or less than an outer diameter of the reducer output shaft 421. With this arrangement, abutment of the inner ring portion 520 against the reducer output shaft 421 may be ensured, thereby reducing or preventing external impurities from entering the receiving space 101 through the gap between the inner wall of the through hole 211 and the reducer output shaft 421.

In one embodiment, a thickness d1 of the inner ring portion 520 is less than a width d2 of the groove 511 of the outer ring portion 510. Alternatively, the thickness d1 of the inner ring portion 520 is less than a thickness d2 of the inner rim of the first circuit board 212 surrounding the through hole 211. In the above configuration, the groove 511 of the outer ring portion 510 has a larger width d2 to increase a contact area between the third sealing member 500" and the first circuit board 212, thereby ensuring a fixed connection therebetween. The thickness d1 of the inner ring portion 520 is relatively small. While ensuring that the inner ring portion 520 abuts against the reducer output shaft 421 to prevent impurities from passing through, excessive heat generated due to relative movement (the reducer output shaft 421 rotates while the third sealing member 500" remains stationary) may be reduced.

In one embodiment, the diameter of the hole 521 may be slightly smaller than the outer diameter of the reducer output shaft, so that when the reducer output shaft passes through the hole 521, the inner ring portion of the third sealing member slightly deforms, thereby providing better sealing. In addition, the thickness d1 of the inner ring portion is relatively small, which helps ensure good sealing while reducing heat generation and ensuring stable operation of the overall structure.

It should be noted that tolerances may occur during machining and assembly of the reducer output shaft 421. In some cases where accumulated tolerances are relatively large, after the reducer 420 or the first encoder 210 is fixed, a clearance between a code disk of the first encoder 210 coupled to the reducer 420 and the first circuit board 212 may become too small to accommodate the dynamic seal structure which, when disposed in the clearance, may result in excessive pressure between the components and thus an excessive axial load on the reducer output shaft 421, causing jamming or even inability of the motor to rotate. While in the solution corresponding to FIGS. 10 and 11, the load is in a radial direction, which may effectively reduce the influence caused by the above tolerances.

In one embodiment, the drive output assembly 300 covers an end of the reducer output shaft 421 facing away from the receiving space 101. In the above configuration, an outer periphery of a portion of the reducer output shaft 421 extending out of the receiving space 101 is wrapped by an integral structure formed by the drive output assembly 300 and the first sealing member 500, thereby effectively reducing or preventing external impurities from entering the receiving space 101 and reducing the risk of damage to components such as the motor 410 and the reducer 420. This is conducive to improving motion accuracy of the instrument drive 43 and the surgical robot 10 and ensuring reliability of surgery.

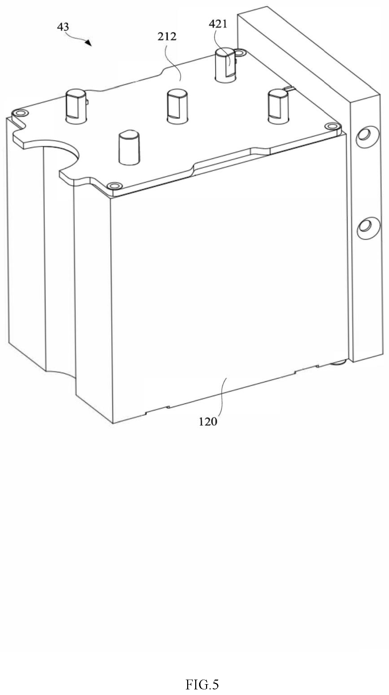

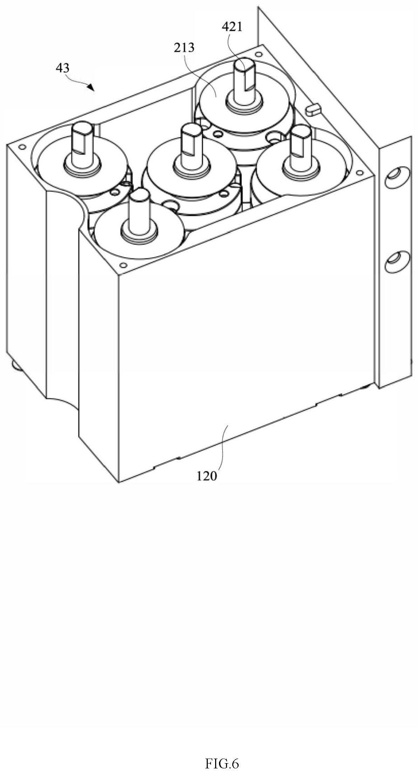

Continuing with FIGS. 3 and 4, and with reference to FIGS. 5 and 6 where necessary, the first encoder stationary portion 214 of the first encoder 210 is fixedly disposed on the first circuit board 212. The first circuit board 212 covers the opening 121 and, together with the carrier 120, defines the receiving space 101. The through hole 211 is located in the first circuit board 212, and the first sealing member 500 abuts against an end of the first circuit board 212 away from the receiving space 101. The first circuit board 212 needs to be provided with a plurality of circuit traces and chip structures, and thus has a relatively large size. Directly using the relatively large first circuit board 212 as a structure for covering the opening 121 may reasonably utilize component structures to form a relatively closed receiving space 101 and prevent or reduce the risk of damage to components such as the motor 410 and the reducer 420 caused by external impurities. At the same time, space utilization is maximized. In some embodiments, the first circuit board may be replaced by another plate-like structure that performs the same function as the first circuit board in closing the opening.

At this time, the first sealing member 500 abuts against the end of the first circuit board 212 away from the receiving space 101, and/or the second sealing member 500' abuts against an end of the first circuit board 212 proximate to the receiving space 101.

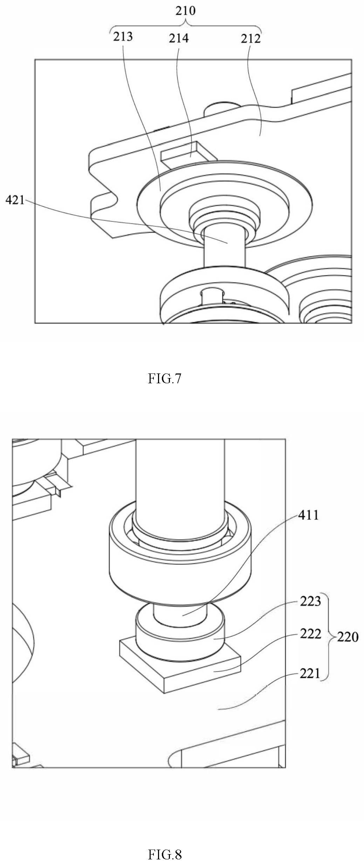

Referring to FIG. 7, the first encoder 210 is a photoelectric encoder. The first encoder stationary portion 214 may include a light source and a photosensitive element, for example a light-signal receiving chip including the photosensitive element. The first encoder rotating portion 213 includes at least a photoelectric code disk. An orthographic projection of the photoelectric code disk onto the first circuit board is larger than the through hole 211. The photoelectric code disk is disposed in the receiving space 101 and fixedly coupled to the reducer output shaft 421, and the light source and the photosensitive element are provided on the first circuit board. The first encoder rotating portion 213 is fixed to the reducer output shaft 421 and rotates synchronously with the reducer output shaft 421. The first encoder stationary portion 214 is fixed on the first circuit board 212 and is electrically coupled to a reading circuit on the first circuit board 212. The first encoder rotating portion 213 outputs an optical signal corresponding to the rotational speed. The first encoder stationary portion 214 receives the corresponding optical signal and converts it into a corresponding electrical signal, and transmits the electrical signal to the first circuit board 212. The first circuit board 212 may further include a controller. The first circuit board 212 may transmit the electrical signal to the controller, and the controller determines the rotational speed of the reducer output shaft 421 based on the electrical signal. With this arrangement, the rotational speed output by the reducer 420 may be monitored in real time to ensure motion accuracy of the instrument drive 43 and the surgical robot 10 and to ensure reliability of surgery. Of course, in other embodiments, the first encoder 210 may alternatively be a magnetoelectric encoder.

In this embodiment, the first encoder rotating portion 213 is arranged in the receiving space 101 and fixed to the reducer 420. With this arrangement, the first encoder rotating portion 213 is disposed in a relatively enclosed space, thereby preventing or reducing external impurities from entering the first encoder rotating portion 213 and ensuring reliability of the first encoder 210. In addition, the first encoder rotating portion 213 and the first circuit board 212 are spaced apart along an axial direction of the reducer output shaft 421 to ensure accuracy of the optical signal.

Referring to FIGS. 5 and 6, in this embodiment, the instrument drive includes a plurality of actuating assemblies, a plurality of drive output assemblies, and a plurality of first encoders. The plurality of actuating assemblies, the plurality of drive output assemblies, and the plurality of first encoders are equal in number and correspond one-to-one. The first circuit board defines a plurality of through holes, and the plurality of through holes correspond to the plurality of first encoders one-to-one. There are multiple first encoders 210 which share the same circuit board. In actual operation, the surgical instrument 44 needs to perform various motions such as yawing, pitching, and gripping, and thus needs to have multiple degrees of freedom. Each degree of freedom requires one drive output assembly 300 and a corresponding first encoder 210. In other words, one surgical instrument 44 uses multiple first encoders 210 to cooperate with each other. When the multiple first encoders 210 share the same circuit board, space may be effectively utilized, which is conducive to simplification and miniaturization of the structure, and the relatively large circuit board may also be used to close the opening 121 of the carrier 120.

Referring to FIGS. 3 and 4, the drive output assembly 300 includes a guide disk 320 and a drive disk 310. The guide disk 320 is fixed to the reducer output shaft 421 and configured to move synchronously with the reducer output shaft 421, and is always disposed in the transmission space 102 defined by the upper housing 110 and the carrier 120. The first sealing member 500 is embedded at an end of the guide disk 320 facing the opening 121. The drive disk 310 is slidably coupled to the drive disk 310 along an axial direction of the reducer output shaft 421, and the drive disk 310 and the guide disk 320 move synchronously in a radial direction. The upper housing 110 defines a through transmission hole 111, and the drive disk 310 may be slidably disposed in the transmission hole 111. In the above configuration, the first sealing member 500 is arranged on the guide disk 320 that always moves synchronously with the reducer output shaft 421, so as to ensure a stable abutting relationship between the first sealing member 500 and the first encoder 210, thereby ensuring that the sealing member 500 provides an effective sealing effect and effectively reducing or preventing external impurities from entering the receiving space 101.

In one embodiment, the drive disk 310 is configured to move axially between a first position 321 and a second position 322, and the first position 321 is closer to the receiving space 101 than the second position 322. When the drive disk 310 is at the first position 321, a minimum distance h1 from the drive disk 310 to the first encoder 210 is not less than an axial dimension of the first sealing member 500. By setting the relationship between the distance from the drive disk 310 to the first encoder 210 and the size of the first sealing member 500, a certain axial clearance between the drive disk 310 and the first encoder 210 may always be ensured, thereby avoiding collisions between the drive disk 310 and the first encoder 210 during movement that could affect detection accuracy of the first encoder 210, and ensuring reliability of the first encoder 210.

Continuing with FIGS. 5 and 6, the encoder assembly 200 further includes a second encoder 220, and the second encoder 220 also includes a second encoder stationary portion and a second encoder rotating portion. The second encoder 220 is disposed in the receiving space 101. The second encoder stationary portion is fixed relative to the carrier 120, and the second encoder stationary portion configured to cooperate with the second encoder rotating portion to detect rotation of an output portion of the actuator.

Specifically, at least a portion of the second encoder 220 is fixed to the motor output shaft 411 of the motor 410, and the second encoder 220 detects rotation of the motor output shaft 411. By providing the second encoder 220 and the first encoder 210 simultaneously, an output rotational speed of the motor 410 and an output rotational speed of the reducer 420 may be monitored in real time, which is conducive to improving motion accuracy and also conducive to monitoring an operating condition of the reducer 420 in real time (e.g., determining whether jamming occurs inside the reducer 420), and to quickly determining whether a foreign matter affects normal operation.

In one embodiment, a rotation axis of the motor output shaft 411 is colinear with a rotation axis of the reducer output shaft 421, and a rotation axis of the first encoder rotating portion is colinear with a rotation axis of the second encoder rotating portion. With this arrangement, space utilization may be further improved and consistency among multiple encoders may be enhanced.

Referring to FIGS. 5 and 6, and with reference to FIG. 8 where necessary, in this embodiment, the second encoder 220 is a magnetoelectric encoder. The second encoder rotating portion includes at least a magnetic element (e.g., a magnet 223), and the second encoder stationary portion includes at least a magnetosensitive element (e.g., a magnetic-signal receiving chip 222). The second encoder stationary portion is spaced apart from the second encoder rotating portion along a rotation axis of the second encoder rotating portion. The second encoder 220 further includes a second circuit board 221, and the magnetic-signal receiving chip 222 and the magnet 223 are arranged in the receiving space 101. In some embodiments, the second circuit board 221 is fixedly provided on the carrier 120, and the second encoder stationary portion is fixedly disposed on the second circuit board. Alternatively, the second circuit board may be understood as being at least part of the second encoder.

The magnet 223 is fixed to the motor output shaft 411 of the motor 410, and the second circuit board 221 is disposed at an end of the magnet 223 away from the first encoder 210. The magnet 223 rotates with the motor output shaft 411 of the motor 410, thereby changing a surrounding magnetic field distribution. The magnetic-signal receiving chip 222 is configured to receive corresponding magnetic signals and convert the magnetic signals into corresponding electrical signals, and transmit the electrical signals to the second circuit board 221. The second encoder 220 may further include a controller. The second circuit board 221 may transmit the electrical signals to the controller, and the controller determines a rotational speed of the motor output shaft 411 of the motor 410 based on the electrical signals. Meanwhile, the second circuit board 221 and the magnet 223 are spaced apart along an axial direction of the reducer output shaft 421, and the magnetic-signal receiving chip 222 and the magnet 223 are also spaced apart along the axial direction of the reducer output shaft 421 to ensure accuracy of the magnetic signals.

In this embodiment, the first encoder is a photoelectric encoder and the second encoder is a magnetoelectric encoder. By configuring the two encoders as different types, interference between multiple encoders may be avoided, thereby improving detection accuracy.

In the present disclosure, the structural embodiments and the method embodiments may supplement each other as long as no conflict exists.

In the present disclosure, the terms "first" and "second" are used for descriptive purposes only and are not to be understood as indicating or implying relative importance. The terms "plurality" and "several" mean two or more, unless expressly defined otherwise.

Those skilled in the art will readily contemplate other embodiments of the present disclosure upon consideration of the specification and practice of the disclosure disclosed herein. The present disclosure is intended to cover any variations, uses, or adaptations of the present disclosure that follow the general principles of the present disclosure and include those departures therefrom that come within known or customary practice in the art. The specification and embodiments are to be regarded as exemplary only, and the true scope and spirit of the present disclosure are indicated by the following claims.

It should be understood that the present disclosure is not limited to the precise structures described above and shown in the drawings, and various modifications and changes may be made without departing from the scope thereof. The scope of the present disclosure is limited only by the appended claims.

## Claims

1. An instrument drive for a surgical robot, the instrument drive comprising:
a carrier defining an opening;
a first circuit board covering the opening and fixed to the carrier, the first circuit board and the carrier enclosing a receiving space; the first circuit board defining a through hole, the through hole communicating the receiving space to an external environment;
an actuating assembly comprising a reducer output shaft, the reducer output shaft being partly disposed in the receiving space and extending out of the receiving space via the through hole, the reducer output shaft being rotatable about an axis of the reducer output shaft;
a drive output assembly coupled to the reducer output shaft, the drive output assembly being rotatable with the reducer output shaft about the axis of the reducer output shaft, the drive output assembly being disposed at an end of the reducer output shaft away from the receiving space;
a first encoder comprising:
a first encoder rotating portion fixed to the reducer output shaft, the first encoder rotating portion being rotatable with the reducer output shaft about the axis of the reducer output shaft, the first encoder rotating portion being disposed in the receiving space;
a first encoder stationary portion being at least a portion of the first circuit board, the first encoder stationary portion configured to cooperate with the first encoder rotating portion to detect rotation of the drive output assembly;
wherein one or more of the drive output assembly, the first encoder rotating portion, or the reducer output shaft is provided with a dynamic seal structure coupled to the first circuit board.

2. The instrument drive of claim 1, wherein the drive output assembly is coupled to a first sealing member, the first sealing member surrounds the drive output assembly and is fixedly embedded in an end of the drive output assembly proximate to the receiving space, an orthographic projection of the first sealing member onto the first circuit board surrounds the through hole, the first sealing member abuts against a surface of the first circuit board facing away from the receiving space, and the first sealing member is kept abutting against the first circuit board in rotation of the first sealing member.

3. The instrument drive of claim 1 or 2, wherein the first encoder rotating portion is coupled to a second sealing member, the second sealing member surrounds the first encoder rotating portion and is fixedly embedded in an end of the first encoder rotating portion proximate to the first circuit board, the second sealing member abuts against the first circuit board, the second sealing member surrounds an outer circumference of the reducer output shaft, an orthographic projection of the second sealing member onto the first circuit board surrounds the through hole, and the second sealing member is kept abutting against the first circuit board in rotation of the second sealing member.

4. The instrument drive of claim 1, wherein the first circuit board is coupled to a third sealing member, the third sealing member is disposed in the through hole, the third sealing member is disposed in a gap between the reducer output shaft and the first circuit board and surrounds the reducer output shaft, and an inner sidewall of the third sealing member abuts against the reducer output shaft.

5. The instrument drive of claim 4, wherein the third sealing member comprises an outer ring portion and an inner ring portion, an outer circumference of the outer ring portion defines a groove, an inner rim of the first circuit board surrounding the through hole is embedded in the groove, the outer ring portion covers the inner rim of the first circuit board surrounding the through hole, the inner ring portion defines a hole, the reducer output shaft passes through the hole, and a diameter of the hole is equal to or less than an outer diameter of the reducer output shaft.

6. The instrument drive of claim 5, wherein a thickness of the inner ring portion is less than a width of the groove, or the thickness of the inner ring portion is less than a thickness of the inner rim of the first circuit board surrounding the through hole.

7. The instrument drive of claim 1, wherein an orthographic projection of the first encoder rotating portion onto the first circuit board is larger than the through hole.

8. The instrument drive of claim 7, wherein the first encoder is a photoelectric encoder, the first encoder rotating portion at least comprises a photoelectric code disk, and an orthographic projection of the photoelectric code disk onto the first circuit board is larger than the through hole.

9. The instrument drive of claim 1 or 7, wherein the first encoder is a photoelectric encoder, the first encoder rotating portion at least comprises a photoelectric code disk, and the encoder stationary portion at least comprises a photosensitive element;
the photoelectric code disk is disposed in the receiving space and fixedly coupled to the reducer output shaft, and a light source and the photosensitive element are provided on the first circuit board.

10. The instrument drive of claim 1 or 5, wherein the instrument drive comprises a plurality of actuating assemblies, a plurality of drive output assemblies, and a plurality of first encoders, the plurality of actuating assemblies, the plurality of drive output assemblies, and the plurality of first encoders are equal in number and correspond one-to-one, the first circuit board defines a plurality of through holes, and the plurality of through holes correspond to the plurality of first encoders one-to-one.

11. The instrument drive of any one of claims 1 to 10, wherein the drive output assembly comprises:
a guide disk fixed to the reducer output shaft and configured to move synchronously with the reducer output shaft; the sealing member being embedded in the guide disk;
a drive disk slidably coupled to the drive disk along an axial direction of the reducer output shaft, and the drive disk and the guide disk being synchronously movable in a radial direction.

12. The instrument drive of claim 11, wherein the drive disk is axially movable between a first position and a second position, the first position is closer to the receiving space than the second position;
when the drive disk is at the first position, a minimum distance from the drive disk to the first circuit board is not less than an axial dimension of the sealing member.

13. The instrument drive of any one of claims 1 to 12, further comprising:
an actuator coupled to a reduction gearbox and configured to actuate the reducer output shaft to rotate;
a second encoder comprising a second encoder rotating portion and a second encoder stationary portion, the second encoder rotating portion coupled to the actuator and configured to be actuated by the actuator to rotate, the second encoder stationary portion fixed relative to the carrier, and the second encoder stationary portion configured to cooperate with the second encoder rotating portion to detect rotation of an output portion of the actuator.

14. The instrument drive of claim 13, wherein the actuator is a motor, the motor comprises a motor output shaft fixed to an input portion of the reduction gearbox and coaxial with the input portion of the reduction gearbox, and the second encoder is configured to detect rotation of the motor output shaft.

15. The instrument drive of claim 14, wherein a rotation axis of the motor output shaft is colinear with a rotation axis of the reducer output shaft, and a rotation axis of the first encoder rotating portion is colinear with a rotation axis of the second encoder rotating portion.

16. The instrument drive of any one of claims 13 to 15, wherein the second encoder is a magnetoelectric encoder, the second encoder rotating portion at least comprises a magnetic element, the second encoder stationary portion at least comprises a magnetosensitive element, and the second encoder stationary portion is spaced apart from the second encoder rotating portion along the rotation axis of the second encoder rotating portion.

17. The instrument drive of claim 16, further comprising a second circuit board fixedly provided on the carrier, wherein the second encoder stationary portion is fixedly disposed on the second circuit board.

18. The instrument drive of any one of claims 1 to 17, wherein the drive output assembly covers an end of the reducer output shaft facing away from the receiving space.

19. A surgical robot, wherein the surgical robot comprises an instrument drive according to any one of claims 1 to 18.
